(19) Europäisches Patentamt
European Patent Office
Office européen des brevets

(11) EP 2 883 454 B1

(12) **EUROPEAN PATENT SPECIFICATION**

(45) Date of publication and mention of the grant of the patent:
**01.08.2018 Bulletin 2018/31**

(21) Application number: **13820278.3**

(22) Date of filing: **18.06.2013**

(51) Int Cl.:
***A01N 43/56** (2006.01)* ***A01N 37/36** (2006.01)*
***A01P 3/00** (2006.01)*

(86) International application number:
**PCT/JP2013/067160**

(87) International publication number:
**WO 2014/013842 (23.01.2014 Gazette 2014/04)**

# (54) COMPOSITION FOR CONTROLLING PLANT DISEASE AND APPLICATION THEREFOR

ZUSAMMENSETZUNG ZUR BEKÄMPFUNG VON PFLANZENBEFALL UND ANWENDUNG DAVON

COMPOSITION PHYTOSANITAIRE ET SON APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2012 JP 2012161709**

(43) Date of publication of application:
**17.06.2015 Bulletin 2015/25**

(73) Proprietor: **Sumitomo Chemical Company Limited
Tokyo 104-8260 (JP)**

(72) Inventor: **MATSUZAKI, Yuichi
Takarazuka-shi
Hyogo 665-8555 (JP)**

(74) Representative: **Vossius & Partner
Patentanwälte Rechtsanwälte mbB
Siebertstrasse 3
81675 München (DE)**

(56) References cited:
**JP-A- 2011 201 850** **JP-A- 2011 246 453**

- **JOURNAL OF PESTICIDE SCIENCE vol. 27, no. 2, 2002, pages 118 - 126, XP008139240**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Technical Field

The present invention relates to a composition for controlling plant disease and an application therefor.

Background Art

Hitherto, some compounds have been developed to control plant disease and have been applied to a practical use (see e.g., Patent Literatures 1 to 4).
Patent Literature 1: WO 86/02641 pamphlet
Patent Literature 2: WO 92/12970 pamphlet
Patent Literature 3: JP 2011 246453 A
Patent Literature 4: JP 2011 201850 A

Moreover, T. Ichiba et al. examine in J. Pesticide Sci., 27, 2002, 118-126 the fungicidal activity of α-alkoxyphenylacetic acid derivatives.

Disclosure of Invention

Technical Problem:

An object of the present invention is to provide a composition having an excellent control efficacy on plant disease.

Technical Solution:

The present inventors have intensively studied to find out a composition having an excellent control efficacy on plant disease. As a result, they have found that a composition comprising a carboxamide compound represented by a formula (I) and a compound represented by a formula (A) shows an excellent control efficacy on plant disease, and therefore the present invention has been completed.
The present invention provides:

[1] A composition for controlling plant disease comprising a carboxamide compound represented by a formula (1):

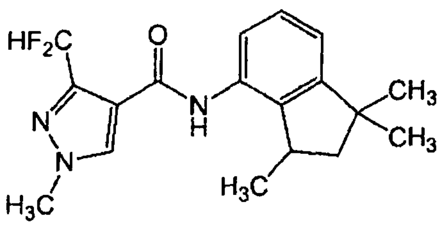
(1)

;

and
a compound represented by a formula (A):

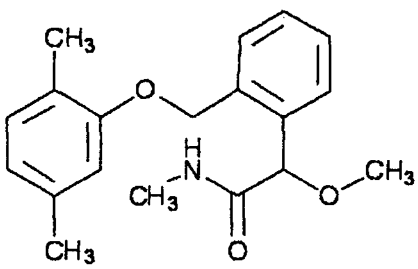
(A)

,

wherein a weight ratio of the carboxamide compound represented by the formula (1) to the compound represented by the formula (A) is that of the carboxamide compound represented by the formula (1)/the compound represented by the formula (A) = 0.1/1 to 10/1.

[2] A method for controlling plant disease comprising applying each of an effective amount of a carboxamide compound represented by a formula (1):

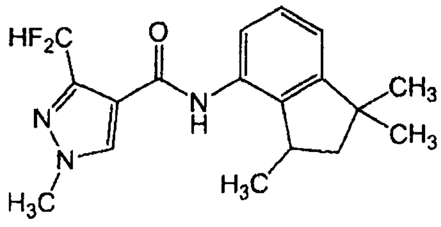

(1)

;

and a compound represented by the formula (A):

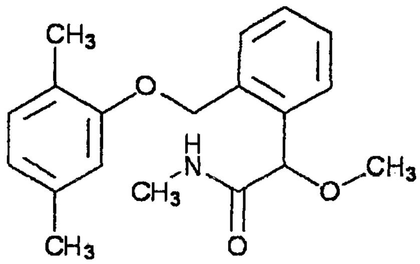

**(A)**

to a plant or a soil for cultivating the plant,
wherein a weight ratio of the carboxamide compound represented by the formula (1) to the compound represented by the formula (A) is that of the carboxamide compound represented by the formula (1)/the compound represented by the formula (A) = 0.1/1 to 10/1.

[3] The method for controlling plant disease described in [2] wherein the plant or the soil for cultivating the plant is soybean or soil for cultivating soybean.

Embodiment for Carrying out the Invention

The composition for controlling plant disease of the present invention (hereinafter, referred to as "Present composition") comprises the carboxamide compound represented by the formula (1) as defined above, which corresponds to a compound represented by the formula (I):

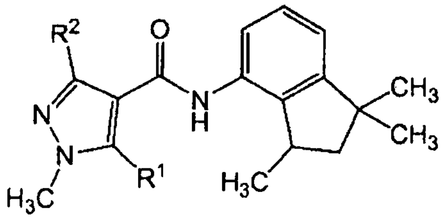

(I)

wherein

$R^1$ represents a hydrogen atom, and
$R^2$ represents a difluoromethyl group

(hereinafter, referred to as "Present carboxamide compound")
and the compound represented by the formula (A):

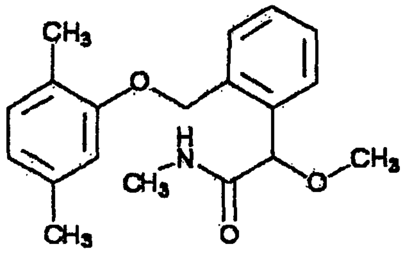

(A)

(hereinafter, referred to as "Present compound (A)").

The present carboxamide compound is described in, for example, WO 86/02641 pamphlet and WO 92/12970 pamphlet, and thus can be prepared according to the method described therein.

The present carboxamide compound has one asymmetric carbon, and due to the asymmetric carbon, there are both enantiomers, that is, the (R) isomer represented by the formula (1-R):

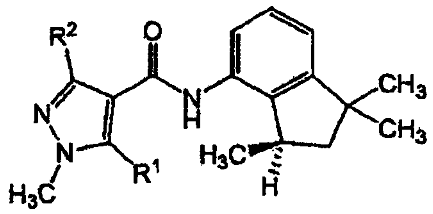

( I-R )

wherein $R^1$ and $R^2$ are the same as defined as above, and the (S) isomer represented by the formula (1-S):

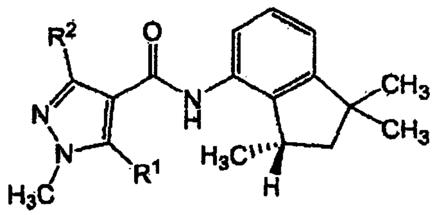

( I-S )

wherein $R^1$ and $R^2$ are the same as defined as above. In the present invention, the present carboxamide compound may be used as any mixture thereof in optional enantiomeric ratio.

A specific example of the present carboxamide compound is a carboxamide compound represented by the formula (1R):

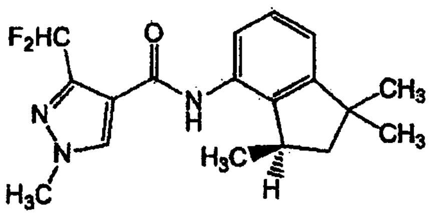

( 1R )

(hereinafter, referred to as "Present carboxamide compound (1R)").

The present compound (A) is a publically known compound and is described, for example, in WO 02/10101 pamphlet. The present compound (A) can be prepared according to the known method.

Herein, the present compound (A) has one asymmetric carbon, and due to the asymmetric carbon, there are both enantiomers, that is, the (R) isomer represented by the formula (A1):

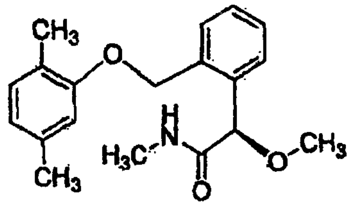

(A1)

,

and

the (S) isomer represented by the formula (A2):

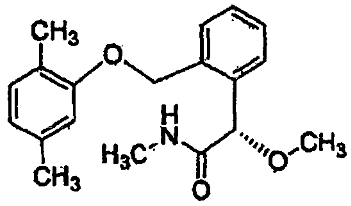

(A2)

.

In the present invention, the present compound (A) may be used as any mixture thereof in optional enantiomeric ratio. The enantiomers can be prepared according to the method described in WO 02/10101 pamphlet.

The weight ratio of the present carboxamide compound to the present compound (A) in the present composition is that of the present carboxamide compound (1)/the present compound (A) = 0.1/1 to 10/1, and preferably 0.5/1 to 1/7.

The present composition is usually prepared by mixing the present carboxamide compound (1), the present compound (A) and an inert carrier, and if necessary, adding a surfactant or other pharmaceutical additives, and then formulating into the form of oil solution, emulsifiable concentrate, flowable formulation, wettable powder, granulated wettable powder, dust formulation, or granules. Such a formulation can be used by itself or with an addition of other inert components as an agent for controlling a plant disease.

Usually, the present composition may contain 0.1 to 99 % by weight, preferably 0.2 to 90 % by weight, and more preferably 1 to 80 % by weight of the present carboxamide compound and the present compound (A) in total.

Examples of a solid carrier used on the formulation include finely-divided power or particles of clay consisting of minerals (for example, kaolin clay, attapulgite clay, bentonite, montmorillonite, acid clay, pyrophyllite, talc, diatomaceous earth, or calcite), natural organic substances (for example, corncob powder, or walnut shell powder), synthetic organic substances (for example, urea), salts (for example, calcium carbonate, or ammonium sulfate), and synthetic inorganic substances (for example, synthetic hydrous silicon oxide).

Examples of a liquid carrier include aromatic hydrocarbons (for example, xylene, alkyl benzene, or methylnaphtalene), alcohols (for example, 2-propanol, ethylene glycol, propylene glycol, or ethylene glycol monoethyl ether), ketones (for example, acetone, cyclohexanone, or isophorone), vegetable oils (for example, soybean oil, or cotton oils), petroleum-derived aliphatic hydrocarbons, esters, dimethylsulfoxide, acetonitrile and water.

Examples of the surfactant include anionic surfactant (for example, alkyl sulfate salts, alkylaryl sulfonate salts, dialkyl sulfosuccinate salts, polyoxyethylene alkylaryl ether phosphates, lignin sulfonate, or naphthalenesulfonate formaldehyde polycondensation), nonionic surfactant (for example, polyoxyethylene alkylaryl ether, polyoxyethylene alkyl polyoxypropylene block copolymer, or sorbitan fatty acid ester) and cationic surfactant (for example, alkyltrimethyl ammonium salts).

Examples of the other pharmaceutical additives include water-soluble polymer (for example, polyvinyl alcohol, or polyvinyl pyrrolidone), polysaccharides (for example, arabic gum, alginic acid and salts thereof, CMC (carboxymethylcellulose), or xanthan gum), inorganic substances (for example, aluminum magnesium silicate, or alumina-sol), antiseptic agent, coloring agent, and PAP (isopropyl acid phosphate), and stabilizing agent (for example, BHT).

The present composition can also be prepared by separately formulating the present carboxamide compound and the present compound (A) into different formulations by the above-mentioned procedures, if necessary, further diluting each of them with water, thereafter, mixing the separately prepared different formulations comprising the present carboxamide compound or the present compound (A), or the dilute solutions thereof with each other.

The present composition can be used to prevent plant from plant disease.

The plant disease which can be controlled by the present composition is exemplified below:

Rice diseases: blast (*Magnaporthe grisea*), brown spot (*Cochliobolus miyabeanus*), sheath blight (*Rhizoctonia solani*), and bakanae disease (*Gibberella fujikuroi*);

Wheat diseases: powdery mildew (*Erysiphe graminis*), fusarium blight (Fusarium gaminearum, *F.* avenaceum, *F.*

*culmorum, Microdochium nivale*), rust (*Puccinia striiformis, P. graminis, P. recondita*), snow mould (*Micronectriella nivale*), typhula snow blight (*Typhula* sp.), loose smut (*Ustilago tritici*), stinking smut (*Tilletia caries*), eyespot (*Pseudocercosporella herpotrichoides*), leaf blotch (*Mycosphaerella graminicola*), glume blotch (*Stagonospora nodorum*), and tan spot (*Pyrenophora tritici-repentis*);

Barley diseases: powdery mildew (*Erysiphe graminis*), loose smut (*Fusarium gaminearum, F. avenacerum, F. culmorum, Microdochium nivale*), rust (*Puccinia striiformis, P. graminis, P. hordei*), loose smut (*Ustilago nuda*), scald (*Rhynchosporium secalis*), net blotch (*Pyrenophora teres*), spot blotch (*Cochliobolus sativus*), leaf stripe (*Pyrenophora graminea*), and damping-off caused by rhizoctonia fungus (*Rhizoctonia solani*);

Corn diseases: smut (*Ustilago maydis*), southern leaf blight (*Cochliobolus heterostrophus*), zonate leaf spot (*Gloeocercospora sorghi*), southern rust (*Puccinia polysora*), gray leaf spot (*Cercospora zeae-maydis*), and damping-off caused by rhizoctonia fungus (*Rhizoctonia solani);*

Citrus diseases: melanose (*Diaporthe citri*), scab (*Elsinoe fawcetti*), Common green mold (*Penicillium digitatum*), blue mold (*P. italicum*), and Phytophthora disease, brown rot (*Phytophthora parasitica, Phytophthora citrophthora*);

Apple diseases: blossom blight (*Monilinia mali*), canker (*Valsa ceratosperma*), powdery mildew (*Podosphaera leucotricha*), Alternaria leaf spot (*Alternaria alternata apple pathotype*), scab (*Venturia inaequalis*), bitter rot (*Colletotrichum acutatum*), and crown rot (*Phytophtora cactorum*);

Pear diseases: scab (*Venturia nashicola, V. pirina*), black spot (*Alternaria alternate* Japanese pear pathotype), rust (*Gymnosporangium haraeanum*), and Phytophthora crown and root rot (*Phytophthora cactorum*);

Peach diseases: brown rot (*Monilinia fructicola*), scab (*Cladosporium carpophilum*), and Phomopsis rot (*Phomopsis* sp.);

Grapes diseases: anthracnose (*Elsinoe ampelina*), ripe rot (*Glomerella cingulata*), powdery mildew (*Uncinula necator*), rust (*Phakopsora ampelopsidis*), black rot (*Guignardia bidwellii*), and downy mildew (*Plasmopara viticola*);

Diseases of Japanese persimmon: anthracnose (*Gloeosporiura kaki*), and leaf spot (*Cercospora kaki, Mycosphaerella nawae*);

Diseases of Cucurbitaceae: anthracnose (*Colletotrichum lagenarium*), powdery mildew (*Sphaerotheca fuliginea*), gummy stem blight (*Mycosphaerella melonis*), Fusarium wilt (*Fusarium oxysporum*), downy mildew (*Pseudoperonospora cubensis*), Phytophthora rot (*Phytophthora* sp.), and damping-off (*Pythium* sp.);

Tomato diseases: early blight (*Alternaria solani*), leaf mold (*Cladosporium fulvum*), and late blight *(Phytophthora infestans);*

Eggplant disease: brown spot (*Phomopsis vexans*), and powdery mildew (*Erysiphe cichoracearum*);

Diseases of brassica family: Alternaria leaf spot (*Alternaria japonica*), white spot (*Cercosporella brassicae*), clubroot (*Plasmodiophora brassicae*), and downy mildew (*Peronospora parasitica*);

welsh onion diseases: rust (*Puccinia allii*), and downy mildew (*Peronospora destructor*);

Soybean diseases: purple stain (*Cercospora kikuchii*), Sphaceloma scad (*Elsinoe glycines*), pod and stem blight (*Diaporthe phaseolorum* var. *sojae*), septoria brown spot (*Septoria glycines*), Cercospora leaf spot (*Cercospora sojina*), rust (*Phakopsora pachyrhizi),* phytophthora root and stem rot *(Phytophthora sojae*), damping-off caused by rhizoctonia fungus (*Rhizoctonia solani*), target spot (*Corynespora casiicola*), and sclerotinia rot (*Sclerotinia sclerotiorum*);

Kindney bean diseases: anthracnose (*Colletotrichum lindemthianum*) ;

Peanut diseases: leaf spot (*Cercospora personata*), brown leaf spot (*Cercospora arachidicola*), and southern blight (*Sclerotium rolfsii*);

Garden pea diseases: powdery mildew (*Erysiphe pisi*);

Potato diseases: early blight (*Alternaria solani*), late blight (*Phytophthora infestans*), pink rot (*Phytophthora erythroseptica*), and powdery scab (*Spongospora subterranean f.* sp. *subterranea*);

Strawberry diseases: powdery mildew (*Sphaerotheca humuli*), and anthracnose (*Glomerella cingulata*);

Tea diseases: net blister blight (*Exobasidium reticulatum*), white scab (*Elsinoe leucospila*), gray blight (*Pestalotiopsis* sp.), and anthracnose *(Colletotrichum theae-sinensis*);

Tabacco diseases: brown spot (*Alternaria longipes*), powdery mildew (*Erysiphe cichoracearum*), anthracnose (*Colletotrichum tabacum*), downy mildew (*Peronospora tabacina*), and black shank (*Phytophthora nicotianae*);

Rape seed diseases: sclerotinia rot *(Sclerotinia sclerotiorum*), and rape seed damping-off caused by Rhizoctonia solani *(Rhizoctonia solani);*

Cotton diseases: cotton damping-off caused by Rhizoctonia solani *(Rhizoctonia solani);*

Sugar beet diseases: cercospora leaf spot (*Cercospora beticola*), leaf blight (*Thanatephorus cucumeris*), root rot (*Thanatephorus cucumeris*), and aphanomyces root rot (*Aphanidermatum cochlioides*);

Rose diseases: blackspot (*Diplocarpon rosae*), powdery mildew (*Sphaerotheca pannosa*), and downy mildew (*Peronospora sparsa*);

Chrysanthemum and Asteraceae vegetable diseases: downy mildew (*Bremia lactucae*), leaf blight (*Septoria chrysanthemi-indici*), and rust (*Puccinia horiana*);

Various plants diseases: diseases caused by Pythium spp. (*Pythium aphanidermatum, Pythium debarianum, Pythium graminicola, Pythium irregulare, Pythium ultimum*), Gray mold (*Botrytis cinerea*), and Sclerotinia rot (*Sclerotinia sclerotiorum*);
Japanese radish diseases: Alternaria leaf spot (*Alternaria brassicicola*);
Turfgrass diseases: dollar spot (*Sclerotinia homeocarpa*), brown patch, and large patch (*Rhizoctonia solani*);
Banana diseases: Sigatoka disease (*Mycosphaerella fijiensis, Mycosphaerella musicola*);
Sunflower diseases: downy mildew (*Plasmopara halstedii*);
Seed diseases or diseases in the early stages of the growth of various plants caused by bacteria of *Aspergillus* spp., *Penicillium* spp., *Fusarium* spp., *Gibberella* spp., *Tricoderma* spp., *Thielaviopsis* spp., *Rhizopus* spp., *Mucor* spp., *Corticium* spp., *Phoma* spp., *Rhizoctonia* spp., *Diplodia* spp.;
Viral diseases of various plants mediated by *Polymixa* genus or *Olpidium* genus.

Examples of the plants to which the composition of the present invention can be applied are as follows:

Crops: corn, rice, wheat, barley, rye, oat, sorghum, cotton, soybean, peanut, buckwheat, beet, rapeseed, sunflower, sugar cane, and tobacco;
Vegetables: solanaceous vegetables (eggplant, tomato, pimento, pepper, and potato), cucurbitaceous vegetables (cucumber, pumpkin, zucchini, water melon, melon, and squash), cruciferous vegetables (Japanese radish, white turnip, horseradish, kohlrabi, Chinese cabbage, cabbage, leaf mustard, broccoli, and cauliflower), asteraceous vegetables (burdock, crown daisy, artichoke, and lettuce), liliaceous vegetables (welsh onion, onion, garlic, and asparagus), ammiaceous vegetables (carrot, parsley, celery, and parsnip), chenopodiaceous vegetables (spinach, and Swiss chard), lamiaceous vegetables (perilla, mint, and basil), strawberry, sweet potato, glutinous yam, and eddoe;
Flowers;
Foliage plants;
Turfgrass;
Fruits: pomaceous fruits (apple, pear, Japanese pear, Chinese quince, and quince), stone fleshy fruits (peach, plum, nectarine, Japanese apricot (*Prunus mume*), cherry fruit, apricot, and prune), citrus fruits (Citrus unshiu, orange, lemon, lime, and grapefruit), nuts (chestnuts, walnuts, hazelnuts, almond, pistachio, cashew nuts, and macadamia nuts), berry fruits (blueberry, cranberry, blackberry, and raspberry), grapes, Japanese persimmon, olive, Japanese plum, banana, coffee, date palm, and coconuts; and
Trees other than fruit trees: tea, mulberry, flowering plants, roadside trees (ash, birch, dogwood, eucalyptus, ginkgo (*ginkgo biloba*), lilac, maple, oak (*quercus*), poplar, Judas tree, Formosan gum (*Liquidambar formosana*), plane tree, zelkova, Japanese arborvitae (*Thuja standishii*), fir wood, hemlock, juniper, pinus, picea, and yew (*Taxus cuspidate*)).

The aforementioned "plants" include plants which resistance has been imparted by genetic recombination.
Among the above, in particular, high control effect against plant diseases that occur in soybeans is expected.
In addition, among plant diseases that occur in these crops, examples of diseases of soybeans to which particularly high control effect is expected include damping-off caused by rhizoctonia fungus (*Rhizoctonia solani*), purple seed stain (*Cercospora kikuchii*), septoria brown spot (*Septoria glycines*), rust (*Phakopsora pachyrhizi*), sclerotinia rot (*Sclerotinia* sclerotiorum) and leaf spot (*Cercospora sojina*).
Exemplary embodiments of the present composition are as follows:

a composition comprising the present carboxamide compound and the present compound (A);
a composition comprising the present carboxamide compound (1R) and the present compound (A);
a composition comprising the present carboxamide compound (1), ethaboxam and the present compound (A);
a composition comprising the present carboxamide compound (1R), ethaboxam and the present compound (A);
a composition comprising the present carboxamide compound (1), metalaxyl and the present compound (A);
a composition comprising the present carboxamide compound (1R), metalaxyl and the present compound (A);
a composition comprising the present carboxamide compound, metalaxyl M and the present compound (A);
a composition comprising the present carboxamide compound (1R), metalaxyl M and the present compound (A);
a composition comprising the present carboxamide compound (1), clothianidin and the present compound (A);
a composition comprising the present carboxamide compound (1R), clothianidin and the present compound (A);
a composition comprising the present carboxamide compound (1), imidacloprid and the present compound (A);
a composition comprising the present carboxamide compound (1R), imidacloprid and the present compound (A);
a composition comprising the present carboxamide compound (1), ipconazole and the present compound (A);
a composition comprising the present carboxamide compound (1R), ipconazole and the present compound (A);

a composition comprising the present carboxamide compound (1), metconazole and the present compound (A);
a composition comprising the present carboxamide compound (1R), metconazole and the present compound (A);
a composition comprising the present carboxamide compound (1), 4-oxo-4-[(2-phenylethyl)amino]-butyric acid and the present compound (A);
a composition comprising the present carboxamide compound (1R), 4-oxo-4-[(2-phenylethyl)amino]-butyric acid and the present compound (A);
a composition comprising the present carboxamide compound (1), tolclofos-methyl and the present compound (A);
a composition comprising the present carboxamide compound (1R), tolclofos-methyl and the present compound (A);
a composition comprising the present carboxamide compound (1), clothianidin, ethaboxam, metalaxyl M and the present compound (A);
a composition comprising the present carboxamide compound (1R), clothianidin, ethaboxam, metalaxyl M and the present compound (A);
a composition comprising the present carboxamide compound (1), imidacloprid, ethaboxam, metalaxyl M and the present compound (A);
a composition comprising the present carboxamide compound (1R), imidacloprid, ethaboxam, metalaxyl M and the present compound (A);

Also in the foregoing exemplary embodiments comprising the present carboxamide compound (1) and the present compound (A), the weight ratio thereof is that of the present carboxamide compound/ the present compound (A) = 0.1/1 to 10/1;
The method for controlling plant diseases of the present invention (hereinafter, referred to as "control method of the present invention") is carried out by applying each of an effective amount of the present carboxamide compound and the present compound (A) to a plant or soil for cultivating the plant. Examples of such plant include foliage of a plant, seeds of a plant and bulbs of a plant. Moreover, the bulbs described herein mean discoid stems, corms, rhizomes, tubers, tuberous, and tuberous roots.
In the control method of the present invention, the present carboxamide compound and the present compound (A) may be applied separately to a plant or soil for cultivating the plant in the same period, but are usually applied as the composition of the present invention in terms of a convenience on applying.
In the control method of the present invention, examples of the method of applying the present carboxamide compound and the present compound (A) include foliage treatment, soil treatment, root treatment and seed treatment.
Such foliage treatment includes, for exmaple, a method of applying the composition of the present invention onto surface of a plant to be cultivated by a foliar application or a stem application.
Such soil treatment includes, for example, soil broadcast, soil incorporation, and irrigation of the medicinal solution to a soil.
Such root treatment includes, for example, a method of soaking a whole or a root of the plant into a medicinal solution comprising the present carboxamide compound and the present compound (A), and a method of attaching a solid formulation comprising the present carboxamide compound, the present compound (A) and the solid carrier to a root of the plant.
Such seed treatment includes, for example, an applying of the composition of the present invention to a seed or a bulb of the plant to be prevented from the plant disease, specifically, for example, spray treatment by spraying a suspension of the composition of the present invention in a mist form onto a surface of a seed or a surface of a bulb, smear treatment by applying the wettable powder, the emulsifiable concentrate or the flowable formulation of the composition of the present invention with added by small amounts of water or as itself to a seed or a bulb, immersion treatment by immersing a seed into a solution of the composition of the present invention for a certain period of time, film-coating treatment by film-coating an admixture of the composition of the present invention and polymer onto a surface of a seed, and pellet-coating treatment by formulating an admixture of the composition of the present invention and powder into a pellet as nuclear seeds.
Each dose of the present carboxamide compound and the present compound (A) in the method for controlling of the present invention may be varied depending on a kind of plant to be treated, a kind or a frequency of an occurrence of a plant disease as a control subject, a dosage form, a treatment period, a treatment method, a treatment site, or a climate condition. In case of an application to a foliage of the plant or a soil for cultivating the plant, a total amount of the present carboxamide compound and the present compound (A) is usually 1 to 500g, preferably 2 to 200g, and more preferably 10 to 100g, per 1000m2. Each dose of the present carboxamide compound and the present compound (A) in the treatment for seed is usually 0.001 to 10g, and preferably 0.01 to 1g, per 1kg of seeds.
The emulsifiable concentrate, the wettable powder or the flowable formulation is usually applied by diluting them with water, and then spreading them. In this case, usually, each concentration of the present carboxamide compound and the present compound (A) contain 0.0005 to 2% by weight, and preferably 0.005 to 1% by weight of the present carboxamide compound and the present compound (A) in total. The dust formulation or the granular formulation is

usually applied as itself without diluting them.

EXAMPLES

Next, the present invention is described in more detail below by Reference Preparation Examples, Formulation Examples and Test Examples.

The Reference Example of the Present carboxamide compound (1R) is indicated below.

Reference Preparation Example 1

To a solution of (R)-1,1,3-trimethyl-4-aminoindane (optical purity 99% ee) 0.15g, triethylamine 0.13g, triethylamine 0.13g, 4-dimethylaminopyridine 5mg and THF 1ml was added dropwise a solution of 1-methyl-3-difluoromethylpyrazol-4-carboxylic acid chloride 0.17g in THF under ice-cooling. The mixture was stirred at room temperature for fifteen minutes, and to the reaction mixture was added ice water and the mixture was extracted with ethyl acetate. The organic layer was washed with saturated aqueous sodium hydrogen carbonate solution and saturated sodium chloride solution successively, dried over magnesium sulfate, and concentrated under reduced pressure. The resulting residue was purified by silica gel column chromatography to give (R)-(-)-N-(1,1,3-trimethylindan-4-yl)-1-methyl-3-difluoromethylpyrazole-4-carboxylic amide (hereinafter, referred to as "Present carboxamide compound") 0.20 g (optical purity 99% ee).

Present carboxamide compound

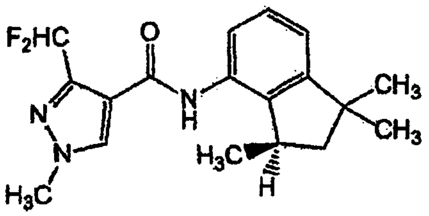

(1R)

$^1$H-NMR ($CDCl_3$) δ: 1.25 (3H, s), 1.28 (3H, d, J = 7.1 Hz), 1.34 (3H, s), 1.67 (1H, dd, J = 12.9, 4.1 Hz), 2.24 (1H, dd, J = 12.9, 8.5 Hz), 3.32-3.41 (1H, m), 3.94 (3H, s), 6.88 (1H, t, J = 54.1 Hz), 6.98 (1H, d, J = 7.6 Hz), 7.22-7.27 (1H, m), 7.79 (1H, d, J = 7.8 Hz), 7.96 (1H, br s), 8.02 (1H, s).

[α] $D^{23}$ = -62° ($CHCl_3$, c 0.99)

Next, the preparation of an intermediate for preparing the present carboxamide compound is indicated.

Reference Preparation Example 2

Using a HPLC method, a racemate of 1,1,3-trimethyl-4-aminoindane 4.8g were separated into each optical isomer thereof under the following condition to obtain (R)-1,1,3-trimethyl-4-aminoindane 1.2g (optical purity 99% ee).

Column: CHIRACEL (registered trademark) OD optically active column
Column temperature: room temperature
Mobile phase: a mixed solvent of hexane and 2-propanol (99:1)
Flow rate: 10mL/minute

(R)-1,1,3-trimethyl-4-aminoindane

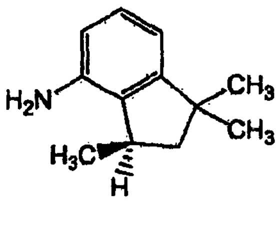

**(*R*-form)**

.

[α] $D^{23}$ = -33.7° ($CHCl_3$, c 0.61)

Reference Preparation Example 3

A racemate of 1,1,3-trimethyl-4-aminoindane 300g, D-tartaric acid 128g and methanol 260 ml were mixed and the mixture was kept at 60°C for 1 hour. Then the mixture was allowed to cool to room temperature, and to the mixture was mixed with about 0.1g of seed crystal, and the mixture was allowed to stand for two days. The formed solids were filterd off and washed with methanol. The resulting solids were recrystallized five times from methanol to afford 1,1,3-trimethyl-4-aminoindane D-tartaric acid salt 100g. To the 1,1,3-trimethyl-4-aminoindane D-tartaric acid salt 78g was added with 5% aqueous sodium hydroxide solution to make pH 10 or more, and the mixture was extracted with methyl t-butylether three times. The resulting oily layer was washed with satrated sodium chloride solution and saturated aqueous sodium hydrogen carbonate solution successfully and then dried over sodium sulfate and concentrated under reduced pressure to give a mixture of 1,13-trimethyl-4-aminoindane 38g in enaniomeric ratio of R isomer /S isomer of 99.6/0.4.

Additionally, the Formulation Examples of the Present composition are indicated. Herein, "parts" means "parts by weight".

Formulation Example 1

Two and a half (2.5) parts of the present carboxamide compound, 1.25 parts of the present compound (A), 14 parts of polyoxyethylene styryl phenyl ether, 6 parts of dodecylbenzene sulfonic acid calcium salt and 76.25 parts of xylene are mixed finely to obtain each formulation.

Formulation Example 2

Two (2) parts of the present carboxamide compound, 8 parts of the present compound (A), 35 parts of a mixture of white carbon and polyoxyethylene alkylether sulfate ammonium salt (weight ratio 1:1), and 55 parts of water are mixed and the resultant solution is then subjected to fine grinding according to a wet grinding method to obtain each formulation.

Formulation Example 3

Five (5) parts of the present carboxamide compound, 10 parts of the present compound (A), 1.5 parts of sorbitan trioleate and 28.5 parts of aqueous solution that contained 2 parts of polyvinyl alcohol are mixed, and the resultant solution is then subjected to fine grinding method, and thereto are added 45 parts of an aqueous solution that contained 0.05 parts of xanthan gum and 0.1 part of aluminum magnesium silicate, followed by adding 10 parts of propylene glycol, and the mixture is blended by stirring to obtain each formulation.

Formulation Example 4

One (1) part of the present carboxamide compound,
4 parts of the present compound (A), 1 part of synthetic hydrous silicon oxide, 2 parts of calcium lignosulfonate, 3 parts of bentonite and 62 parts of kaolin clay are fully ground and mixed, and the resultant mixture is added with water and fully kneaded, and then subjected to granulation and drying to obtain each formulation.

Formulation Example 5

Twelve and a half (12.5) parts of the present carboxamide compound, 37.5 parts of the present compound (A), 3 parts of calcium lignosulfonate, 2 parts of sodium lauryl sulfate and 45 parts of synthetic hydrous silicon oxide are fully ground and mixed to obtain each formulation.

Formulation Example 6

Three (3) parts of the present carboxamide compound,
2 parts of the present compound (A), 85 parts of kaolin clay and 10 parts of talc are fully ground and mixed to obtain each formulation.

Further, Test Examples are indicated.

Test Example 1

One hundred (100) μl of cyclohexane solution containing a predetermined weights of a testing compound was applied on 10g of soybean seeds (cultivar; natto-shoryu) using a rotary seed treatment machine (seed dresser, produced

by Hans-Ulrich Hege GmbH).

One (1) day after the above treatment, a plastic pot was filled with soils contaminated with rhizoctonia fungus (*Rhizoctonia solani*), and the seeds treated with the testing compound were sown and grown in a glass greenhouse for twenty days (which is defined as "treated group"). Thereafter, the presence or absence of onset of damping-off caused by rhizoctonia fungus (*Rhizoctonia solani*) was observed in nursery plant germinated from each seed and the incidence rate was then calculated by the following equation (1).

Separately, soybean seeds without the above seed treatment were grown similarly to the treated group (which is defined as "untreated group"). The incidence rate was calculated similarly to the treated group.

Here, the carboxamide compound represented by formula (1) used in Test Example 1 and the present compound (A) used in Test Example 1 were used in racemic mixture respectively.

From each of the incidence rates of the treated group and the untreated group, the efficacy of the treated group was calculated by the following equation (2). The results are shown in Table 1.

Incidence rate (%) = the number of germinated nursery plants / the total number of the nursery plants × 100 (Equation (1))

Efficacy (%) = (1- incidence rate of the treated group / incidence rate of the untreated group) × 100 (Equation (2))

[Table 1]

| Present carboxamide compound [g/100kg seeds] | Present compound (A) [g/100kg seeds] | Efficacy (%) |
|---|---|---|
| 2 | 2 | 100 |

Test Example 2

A plastic pot was filled with soil and thereto soybeans (cultivar; natto-shoryu) was seeded and the plants were grown in a greenhouse for fourteen (14) days. A testing compound was dissolved in a CEC cocktail (cyclohexane : Sorpol (trademark) 2680X (Toho Chemical Industry Co. Ltd.) = 5:1 (volume ratio)), and the resulting mixture was formulated into an emulsifiable concentrate containing the testing compound by 5% (w/v) in total and diluted with water to a predetermined concentration, and the diluted solution was applied by sprayer onto soybeans to adhere fully onto the surfaces of leaves of the above soybeans. After spraying, the plants were air-dried and thereto an aqueous suspension of soybeans rust (*Phakopsora pachyrhizi*) urediniospore (about 10,000 / mL) was inoculated by spraying 1 day after the treatment.

In early times after the inoculation, the plants were cultivated at 20 to 23°C under humid condition for 1 day, and cultivated in a greenhouse for additional ten 10 days (hereinafter referred to as "treated group"). Thereafter, the lesion area of soybeans rust (*Phakopsora pachyrhizi*) was assessed.

Whereas, soybeans were cultivated similarly to the treated group except of no foliage application of the above diluted solution of the testing compound (hereinafter referred to as "untreated group"). The lesion area of soybeans rust (*Phakopsora pachyrhizi*) was assessed similarly to the treated group.

Here, the carboxamide compound represented by formula (1) used in Test Example 2 and the compound represented by formula (A) used in Test Example 2 were used in racemic mixture respectively.

From each of the lesion area of the treated group and the untreated group, the efficacy of the treated group was calculated by the following equation (3). The results are shown in Table 3.

Efficacy (%) = (1- lesion area of the treated group / lesion area of the untreated group) × 100 (Equation (3))

[Table 3]

| Present carboxamide compound [ppm] | Present compound (A) [ppm] | Efficacy (%) |
|---|---|---|
| 2 | 10 | 100 |

Test Example 3

One hundred (100) μl of cyclohexane solution containing a predetermined weights of a testing compound is applied on 10g of soybean seeds (cultivar; natto-shoryu) using a rotary seed treatment machine (seed dresser, produced by Hans-Ulrich Hege GmbH).

One (1) day after the above treatment, a plastic pot is filled with soils contaminated with rhizoctonia fungus (*Rhizoctonia solani*), and the seeds treated with the testing compound are sown and grown in a glass greenhouse for twenty days (which is defined as "treated group"). Thereafter, the presence or absence of onset of damping-off caused by rhizoctonia fungus (*Rhizoctonia solani*) is observed in nursery plant germinated from each seed and the incidence rate is then calculated by the following equation (4) .

Separately, soybean seeds without the above seed treatment are grown similarly to the treated group (which is defined as "untreated group"). The incidence rate is calculated similarly to the treated group.

Herein, the carboxamide compound represented by formula (I) used in Test Example 3 is used in a mixture of R isomer : S isomer of 80:20 to 100:0, more preferably R isomer : S isomer of 95:5 to 99:1. Also, the present compound (A) used in Test Example 3 is used in racemic mixture.

From each of the incidence rates of the treated group and the untreated group, the efficacy of the treated group is calculated by the following equation (5).

Incidence rate (%) = the number of germinated nursery plants / the total number of the nursery plants × 100 (Equation (4))

Efficacy (%) = (1- incidence rate of the treated group / incidence rate of the untreated group) × 100 (Equation (5))

[Industrial Applicability]

The present invention enables plant to prevent from plant diseases.

**Claims**

1. A composition for controlling plant disease comprising a carboxamide compound represented by a formula (1):

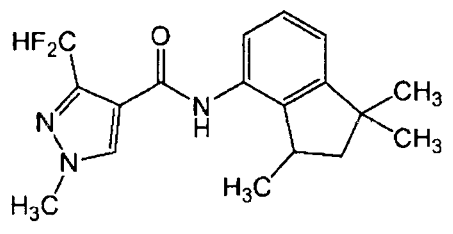

(1)

;

and
a compound represented by a formula (A):

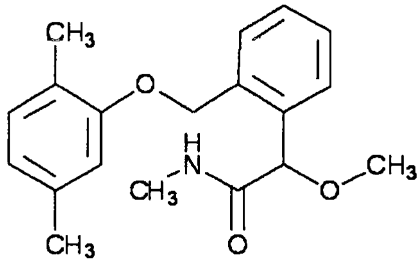

(A)

,

wherein a weight ratio of the carboxamide compound represented by the formula (1) to the compound represented by the formula (A) is that of the carboxamide compound represented by the formula (1)/the compound represented by the formula (A) = 0.1/1 to 10/1.

**2.** A method for controlling plant disease comprising applying each of an effective amount of a carboxamide compound represented by a formula (1):

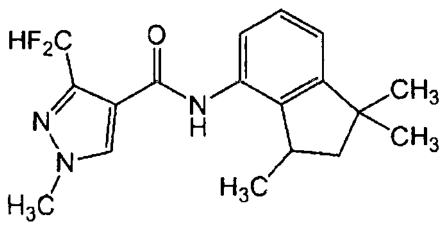

(1)

;

and a compound represented by the formula (A):

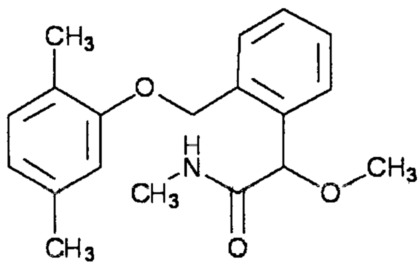

(A)

to a plant or a soil for cultivating the plant,
wherein a weight ratio of the carboxamide compound represented by the formula (1) to the compound represented by the formula (A) is that of the carboxamide compound represented by the formula (1)/the compound represented by the formula (A) = 0.1/1 to 10/1.

**3.** The method for controlling plant disease according to claim 2, wherein the plant or the soil for cultivating the plant is soybean or soil for cultivating soybean.

**Patentansprüche**

**1.** Eine Zusammensetzung zur Bekämpfung von Pflanzenbefall, umfassend eine Carboxamidverbindung dargestellt durch eine Formel (1):

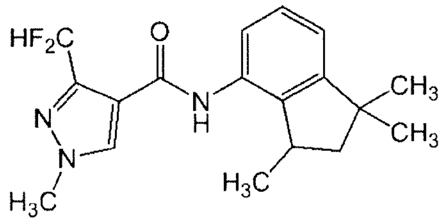

(1)

;

und

eine Verbindung, dargestellt durch eine Formel (A):

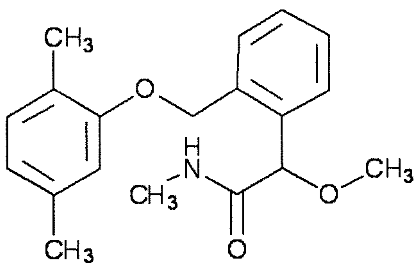

(A)

,

wobei ein Gewichtsverhältnis der Carboxamidverbindung dargestellt durch die Formel (1) zu der Verbindung dargestellt durch die Formel (A) das der Carboxamidverbindung dargestellt durch die Formel (1)/der Verbindung dargestellt durch die Formel (A) = 0,1/1 bis 10/1 beträgt.

**2.** Ein Verfahren zur Bekämpfung von Pflanzenbefall, umfassend das Aufbringen jeweils einer wirksamen Menge einer Carboxamidverbindung dargestellt durch eine Formel (1):

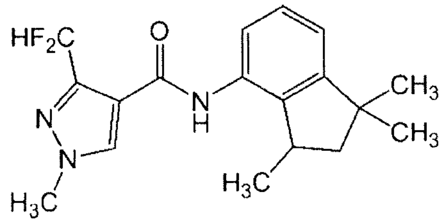

(1)

;

und einer Verbindung dargestellt durch die Formel (A):

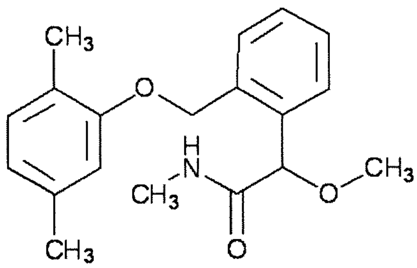

(A)

auf eine Pflanze oder einen Boden zum Anbau der Pflanze,
wobei ein Gewichtsverhältnis der Carboxamidverbindung dargestellt durch die Formel (1) zu der Verbindung dargestellt durch die Formel (A) das der Carboxamidverbindung dargestellt durch die Formel (1)/der Verbindung dargestellt durch die Formel (A) = 0,1/1 bis 10/1 beträgt.

**3.** Das Verfahren zur Bekämpfung von Pflanzenbefall gemäß Anspruch 2, wobei die Pflanze oder der Boden zum Anbau der Pflanze Sojabohne oder Boden zum Anbau von Sojabohnen ist.

## Revendications

**1.** Composition pour lutter contre une maladie des plantes comprenant un composé de carboxamide représenté par une formule (1) :

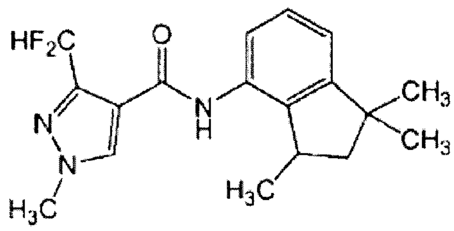

(1)

;

et un composé représenté par une formule (A) :

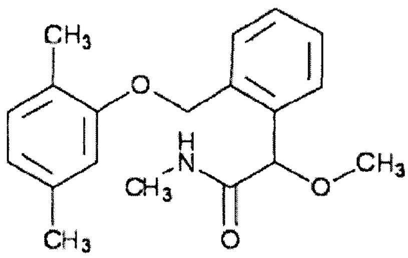

(A)

,

où un rapport en poids du composé de carboxamide représenté par la formule (1) au composé représenté par la formule (A) est celui de le composé de carboxamide représenté par la formule (1)/le composé représenté par la formule (A) = 0,1/1 à 10/1.

**2.** Procédé pour lutter contre une maladie des plantes comprenant l'application de chacune d'une quantité efficace d'un composé de carboxamide représenté par une formule (1) :

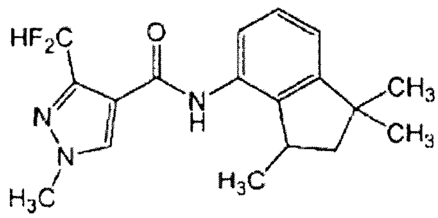

(1)

;

et d'un composé représenté par la formule (A) :

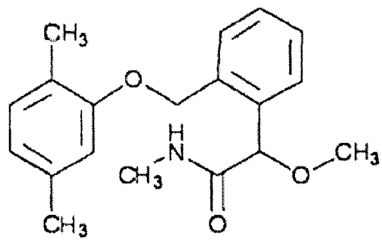

(A)

à une plante ou un sol pour cultiver la plante,
où un rapport en poids du composé de carboxamide représenté par la formule (1) au composé représenté par la formule (A) est celui de le composé de carboxamide représenté par la formule (1)/le composé représenté par la formule (A) = 0,1/1 à 10/1.

3. Procédé pour lutter contre une maladie des plantes selon la revendication 2, où la plante ou le sol pour cultiver la plante est le soja ou un sol pour cultiver le soja.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 8602641 A
- WO 9212970 A
- JP 2011246453 A
- JP 2011201850 A
- WO 0210101 A


### Non-patent literature cited in the description


- **MOREOVER, T. ICHIBA et al.** *J. Pesticide Sci.,* 2002, vol. 27, 118-126